# EUROPEAN PATENT SPECIFICATION

(11) **EP 3 597 146 B1**
(45) Date of publication and mention of the grant of the patent: **03.08.2022**
(21) Application number: 18768570.6
(22) Date of filing: 09.03.2018
(51) Int. Cl.: A61C 19/04, G01B 11/24, A61C 9/00, A61B 5/00

(54) **DENTAL MEASURING DEVICE AND DENTAL MEASURING SYSTEM**
DENTALE MESSVORRICHTUNG UND DENTALES MESSSYSTEM
DISPOSITIF DE MESURE DENTAIRE ET SYSTÈME DE MESURE DENTAIRE

(30) Priority: 14.03.2017 JP 2017048962
(43) Date of publication of application: 22.01.2020
(73) Proprietor: J. Morita MFG. Corp., Kyoto-shi, Kyoto 612-8533 (JP)
(72) Inventor: TSUDA, Keiichi, Kyoto-shi Kyoto 612-8533 (JP); NISHIMURA, Mikinori, Kyoto-shi Kyoto 612-8533 (JP); SORIMOTO, Keisuke, Kyoto-shi Kyoto 612-8533 (JP)
(74) Representative: Müller Hoffmann & Partner
(86) International application number: PCT/JP2018/009173
(87) International publication number: WO 2018/168681

(56) References cited:
- JP-A- 2005 164 435
- JP-A- 2009 297 392
- US-A1- 2003 165 794
- US-A1- 2008 293 008
- US-A1- 2016 256 054

## Description

### TECHNICAL FIELD

The present invention relates to a dental measurement apparatus, a tip unit of the dental measurement apparatus that can be attached to a main body unit of the dental measurement apparatus, and a dental measurement system including the dental measurement apparatus and a management apparatus configured to manage management information used in calibration in the dental measurement apparatus.

### BACKGROUND ART

In the dental field, a measurement apparatus configured to measure a three-dimensional shape of a tooth has been developed for treatment or diagnosis of the tooth (Japanese Patent Laying-Open No. 2016-166765). A hand-held scanner disclosed in Japanese Patent Laying-Open No. 2016-166765 as one example of the measurement apparatus includes a tip unit that can be inserted into an oral cavity. The hand-held scanner is configured such that the light having a linear pattern (hereinafter, also referred to as "pattern"), which has passed through a mirror provided in the tip unit, is projected onto an object to be measured, and an image pickup unit picks up an image of the reflected light from the object to be measured that has passed through the mirror.

The tip unit that may come into contact with a living body as a result of insertion into an oral cavity must be subjected to sterilization treatment for infection control, when the tip unit is used for measurement and then used for a subject different from a subject who is a target of the measurement. Therefore, there is a hand-held scanner configured such that only a tip unit that may come into contact with a living body can be removed and replaced.

US2016/256054 discloses 3D optical scanners for scanning surfaces in humid environments and/or in environments with high requirements to hygiene, such as in body orifices, where condensation on optical surfaces is likely to occur.

US2003165794 relates to an identification type instrument assembly detachably connected to the main body of a medical apparatus for use in diagnosis and treatment, an identification type adapter and an identification type tube connecting an instrument and the main body, and medical apparatus using them in medical or dental field.

### CITATION LIST

### PATENT DOCUMENT

PTD 1: Japanese Patent Laying-Open No. 2016-166765

### SUMMARY OF INVENTION

### TECHNICAL PROBLEM

However, each optical element which is a mirror has an individual difference such as a difference in reflectivity and a difference in warpage of the mirror. In order to obtain an accurate measurement value, it is preferable to perform calibration for each optical element. However, in the case of performing calibration for each optical element, it is necessary to obtain a calibration parameter every time a tip unit is replaced and a different optical element is used, which may lead to complication of a measurement flow.

The present invention has been made to solve the above-described problem and an object of the present invention is to provide a dental measurement apparatus, a tip unit of the dental measurement apparatus, and a dental measurement system, which make it possible to obtain an accurate measurement value in consideration of an influence of each individual optical element.

### SOLUTION TO PROBLEM

The invention is as defined in the appended claims.

A dental measurement apparatus according to the present invention includes: a main body unit configured to measure an object to be measured; and a tip unit provided so as to be attachable and detachable to and from the main body unit. The tip unit includes: an optical element configured to be capable of introducing light from the object to be measured into the main body unit for measurement in the main body unit; and identification information for identifying the tip unit individually. For use in calibration in the dental measurement apparatus, the main body unit is capable of obtaining management information about an individual difference of the tip unit caused by the optical element, based on the identification information.

A tip unit of a dental measurement apparatus according to the present disclosure is provided so as to be attachable and detachable to and from a main body unit. The tip unit includes: an optical element configured to be capable of introducing light from an object to be measured into the main body unit for measurement in the main body unit; and identification information for identifying the tip unit individually. The tip unit allows the main body unit to, for use in calibration in the dental measurement apparatus, obtain management information about an individual difference of the tip unit caused by the optical element, based on the identification information.

A dental measurement system according to the present invention includes: a dental measurement apparatus; and a management apparatus configured to manage management information used in calibration in the dental measurement apparatus. The dental measurement apparatus of the dental measurement system includes: a main body unit configured to measure an object to be measured; and a tip unit provided so as to be attachable and detachable to and from the main body unit. The tip unit of the dental measurement apparatus includes: an optical element configured to be capable of introducing light from the object to be measured into the main body unit for measurement in the main body unit; and identification information for identifying the tip unit individually. The management apparatus of the dental measurement system includes a storage unit configured to store an individual difference of the tip unit caused by the optical element as the management information in association with the identification information. For use in calibration in the dental measurement apparatus, the main body unit of the dental measurement apparatus is capable of obtaining the management information about the individual difference of the tip unit caused by the optical element.

### ADVANTAGEOUS EFFECTS OF INVENTION

For use in calibration in the dental measurement apparatus, the dental measurement apparatus according to the present invention can obtain the management information about the individual difference of the tip unit caused by the optical element, based on the identification information, and thus, can obtain an accurate measurement value in consideration of an influence of each individual optical element. In addition, the tip unit of the dental measurement apparatus according to the present disclosure allows the main body unit to obtain the management information about the individual difference of the tip unit caused by the optical element, based on the identification information, and thus, can obtain an accurate measurement value in consideration of an influence of each individual optical element by using the tip unit of the dental measurement apparatus according to the present invention. For use in calibration in the dental measurement apparatus, the dental measurement system according to the present invention obtains the management information about the individual difference of the tip unit caused by the optical element, and thus, can obtain an accurate measurement value in consideration of an influence of each individual optical element.

### BRIEF DESCRIPTION OF DRAWINGS

Fig. 1 shows an overview of a configuration of a dental measurement system according to a first embodiment of the present invention.
Fig. 2 is a schematic view of a configuration of a probe according to the first embodiment of the present invention.
Fig. 3 is a block diagram for illustrating the dental measurement system according to the first embodiment of the present invention.
Fig. 4 is a flowchart showing a measurement process executed by a control unit of an intraoral scanner according to the first embodiment of the present invention.
Fig. 5 is a flowchart showing the measurement process executed by the control unit of the intraoral scanner according to the first embodiment of the present invention.
Fig. 6 is a flowchart showing a sterilization information update process executed by a control unit of a sterilizer according to the first embodiment of the present invention.
Fig. 7 is a schematic view showing a process executed by the dental measurement system according to the first embodiment of the present invention.
Fig. 8 is a block diagram for illustrating an intraoral scanner according to a second embodiment of the present invention.
Fig. 9 is a block diagram for illustrating an intraoral scanner according to a third embodiment of the present invention.
Fig. 10 is one example of a flowchart showing a process executed by a dental measurement system according to a fourth embodiment of the present invention, when there is no management data corresponding to identification data.

### DESCRIPTION OF EMBODIMENTS

Hereinafter, embodiments according to the present invention will be described with reference to the drawings.

### (First Embodiment)

A dental measurement system 100 according to a first embodiment of the present invention is a system used in an intraoral scanner 200 configured to measure a three-dimensional shape of a tooth in an oral cavity. However, dental measurement system 100 according to the present invention is not limited to the system used in intraoral scanner 200 and is applicable to a system having a similar configuration and configured to measure the properties of a tooth different from the three-dimensional shape. For example, dental measurement system 100 according to the present invention is applicable to a system of an optical interference tomographic apparatus configured to measure a cross-sectional shape of a tooth, and systems of a spectrometer and a colorimeter configured to measure the color of a tooth.

### [Configuration of Dental Measurement System]

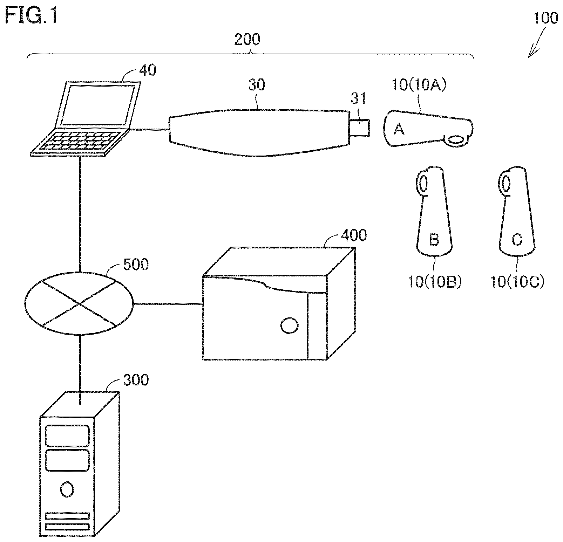

Fig. 1 shows an overview of a configuration of dental measurement system 100 according to the first embodiment of the present invention. Dental measurement system 100 shown in Fig. 1 includes intraoral scanner 200, a server 300, a sterilizer 400, and a network 500 including the Internet and others (hereinafter, also referred to as "network 500"). Each of intraoral scanner 200, server 300 and sterilizer 400 is connected to network 500 and can mutually communicate with other devices connected to network 500.

Intraoral scanner 200 includes a probe 10, an optical measurement unit 30, a control device 40, and a not-shown power supply unit. Probe 10 is a tip unit provided so as to be attachable and detachable to and from optical measurement unit 30, and guides the reflected light from an object to be measured in an oral cavity to optical measurement unit 30. Optical measurement unit 30 is a main body unit configured to measure the object to be measured, and irradiates the object to be measured with light via probe 10 and detects the reflected light from the object to be measured that has been guided by probe 10. Control device 40 controls the operation of optical measurement unit 30, and obtains a three-dimensional shape of the object to be measured based on the reflected light detected by optical measurement unit 30.

Specifically, optical measurement unit 30 projects the light having a pattern (hereinafter, also referred to as "pattern") onto the object to be measured via probe 10. Probe 10 guides, to optical measurement unit 30, the reflected light from the object to be measured having the pattern projected thereon. Optical measurement unit 30 detects the reflected light from the object to be measured that has been guided by probe 10. Control device 40 obtains a three-dimensional shape based on the light detected by optical measurement unit 30. Control device 40 can measure the three-dimensional shape using principles such as, for example, a confocal method, a triangulation method, a focusing method, white light interferometry, a stereo method, a photogrammetry method, an SLAM method (Simultaneous Localization and Mapping), and optical coherence tomography (OCT). In the case of using a principle that allows measurement of the three-dimensional shape by projecting the uniform light having no pattern, optical measurement unit 30 may project the uniform light onto the object to be measured.

### [Configuration of Probe]

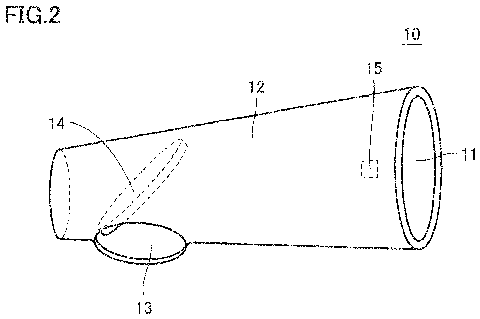

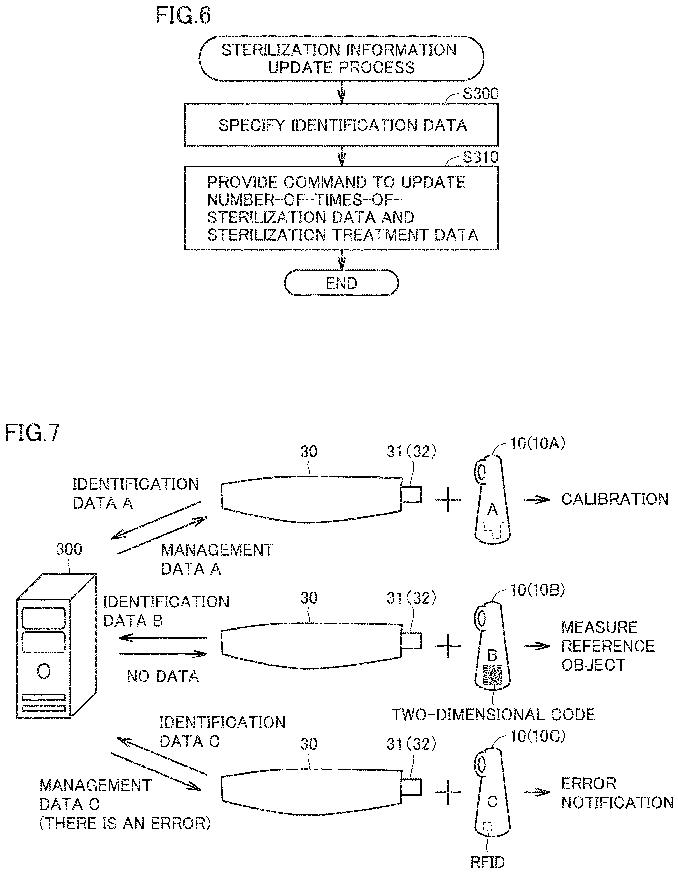

Fig. 2 is a schematic view of a configuration of probe 10 according to the first embodiment of the present invention. Although probe 10 has a cylindrical shape in Fig. 2, the shape of probe 10 is not limited thereto. For example, probe 10 may have a stereoscopic shape having a rectangular cross section or a chamfered polygonal cross section. Probe 10 includes a housing 12 having an opening 11 for connection to optical measurement unit 30, a measurement window 13 (light introduction unit) provided in housing 12 on the opposite side of opening 11, and a mirror which is an optical element 14 configured to reflect the light introduced from measurement window 13 in a direction of opening 11. Opening 11 is an insertion portion through which a connection unit 31 (see Fig. 7) of optical measurement unit 30 is inserted. In addition, identification data 15 for identifying probe 10 individually is provided at a position of opening 11 that is in proximity to or in contact with connection unit 31 when optical measurement unit 30 and probe 10 are connected.

Probe 10 is attachable and detachable to and from optical measurement unit 30. Therefore, for infection control, only probe 10 that may come into contact with a living body can be removed from optical measurement unit 30 and be subjected to sterilization treatment (e.g., treatment in high-temperature and high-humidity environment). The sterilization treatment can be performed using sterilizer 400.

For infection control, probe 10 is replaced every time probe 10 is used. For example, when a measurer such as a dentist uses a probe 10A for a subject such as a patient and then uses intraoral scanner 200 for a different subject, the measurer can remove probe 10A from optical measurement unit 30 and attach unused probe 10 such as a probe 10B or a probe 10C to optical measurement unit 30.

Identification data 15 is data that can identify probe 10 individually. For example, probe 10A is provided with identification data 15A that can identify probe 10A, and similarly, probe 10B is provided with identification data 15B and probe 10C is provided with identification data 15C. In addition, optical measurement unit 30 that can be connected to probe 10 includes a reading unit 32 configured to read identification data 15. Reading unit 32 reads identification data 15, and thereby, optical measurement unit 30 or control device 40 communicably connected to optical measurement unit 30 specifies which one of probe 10A to probe 10C probe 10 is, for example.

### [Configuration of Optical Measurement Unit and Control Device]

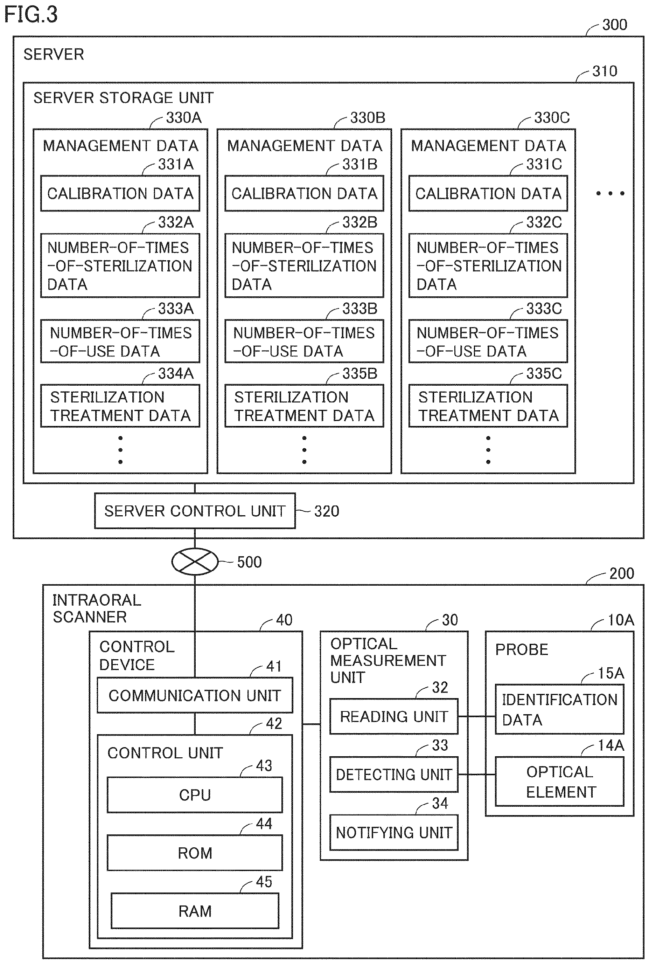

A configuration of optical measurement unit 30 and control device 40 of intraoral scanner 200 will be described in detail with reference to Fig. 3. Fig. 3 is a block diagram for illustrating dental measurement system 100 according to the first embodiment of the present invention. In Fig. 3, sterilizer 400 is not shown in order to describe an overview of dental measurement system 100.

Optical measurement unit 30 includes connection unit 31 (see Fig. 7) that can be fitted into opening 11 of probe 10, reading unit 32 configured to read identification data 15 of probe 10, a detecting unit 33 configured to detect the reflected light from the object to be measured that has been guided by probe 10, and a notifying unit 34 configured to provide notification of information about intraoral scanner 200 including probe 10. Although not shown, optical measurement unit 30 also has an optical component and a light source configured to generate the pattern projected onto the object to be measured, a lens component configured to form an image of the pattern on a surface of the object to be measured, a focus adjustment mechanism configured to adjust a focus of the lens, a converter configured to convert information (actual measurement value) about the reflected light detected by detecting unit 33 to electrical information, a transmission and reception unit (not shown) configured to be communicable with control device 40, and the like. The transmission and reception unit transmits the electrical information converted by the converter and identification data 15 read by reading unit 32 to control device 40, and receives information transmitted from control device 40. Detecting unit 33 may, for example, be an image pickup element or the like configured to pick up an image of the projected pattern, and may be any element as long as detecting unit 33 detects the data for obtaining the three-dimensional shape.

In dental measurement system 100 according to the first embodiment of the present invention, reading unit 32 is provided in connection unit 31 (see Fig. 7). When probe 10 and optical measurement unit 30 are connected, reading unit 32 comes closer to identification data 15 of opening 11 and thus reading unit 32 can read identification data 15. That is, when probe 10 is connected to optical measurement unit 30 at the time of measurement, reading unit 32 can read identification data 15. As a result, there is no need to add the additional work of causing reading unit 32 to read identification data 15, and thus, complication of a measurement flow can be prevented. In addition, there is no need to manually input identification data 15, and thus, contamination of surrounding instruments with the measurer's hand having touched an oral cavity can be prevented.

Reading unit 32 may only be capable of reading identification data 15, and a position where reading unit 32 is provided is not particularly limited and a reading method is not particularly limited, either.

For example, the shape of opening 11 may be designed differently for each of probes 10A to 10C and reading unit 32 may read identification data 15 by specifying the shape of opening 11. In such a case, shape data specific to each probe 10 corresponds to identification data 15. With such a configuration, probe 10 can be provided with identification data 15, simply by changing the shape of opening 11 of probe 10. A position where the shape specific to each probe 10 is provided is not limited to opening 11, and the shape of at least a part of probe 10 may be the shape specific to each probe 10.

In addition, an optically recognizable tag may be provided for each of probes 10A to 10C and reading unit 32 may read identification data 15 by optically recognizing the tag. Examples of the optically recognizable tag include a one-dimensional code such as a barcode, a two-dimensional code such as a QR code (registered trademark), a code formed of a combination of color, and the like. Probe 10 can be provided with the optically recognizable tag, simply by printing the code on probe 10. Examples of a method for printing the code include screen printing, sputtering, vapor deposition, laser marking and the like, although any printing method may be used. Probe 10 may also be provided with the tag, by attaching a member having the code printed thereon to probe 10. Examples of an attaching method include attachment by an adhesive, attachment by a mechanical fitting structure, and the like, although any attachment method may be used.

In addition, an electromagnetically recognizable tag may be provided for each of probes 10A to 10C and reading unit 32 may read identification data 15 by electromagnetically recognizing the tag. Examples of the electromagnetically recognizable tag include a passive tag without a built-in battery and an active tag with a built-in battery, which are used in an RFID (Radio frequency identifier), a recording medium in which data is stored in accordance with a magnetization pattern, a recording medium in which data is stored in accordance with a magneto-optical method, and the like. In the case of reading identification data 15 using the electromagnetically recognizable tag as described above, probe 10 can be provided with identification data 15, simply by embedding the tag in probe 10. In the case of using the passive tag, there is no built-in battery, and thus, the size is small and the passive tag can be easily provided in probe 10.

Control device 40 includes a communication unit 41 and a control unit 42. Control unit 42 controls the operation of optical measurement unit 30, and obtains the three-dimensional shape based on the reflected light detected by optical measurement unit 30. Control unit 42 is provided with a CPU 43 (Central Processing Unit) serving as a control center, an ROM 44 (Read Only Memory) storing a program, control data and the like for causing the CPU to operate, an RAM 45 (Random Access Memory) functioning as a work area of CPU 43, an input and output interface (not shown) configured to maintain the signal integrity with peripheral devices such as optical measurement unit 30, and the like. Communication unit 41 is connected to network 500 and communicates with other computers (e.g., server 300) connected to network 500.

Control unit 42 calibrates a measurement distortion caused by optical element 14 of probe 10 and obtains the three-dimensional shape. Control unit 42 also executes a process for updating management data 330 managed for each probe 10. Control unit 42 also executes a process for measuring a calibration parameter used for calibration performed during obtainment of the three-dimensional shape.

Management data 330 managed for each probe 10 is stored in server 300. Management data 330 includes calibration data 331, number-of-times-of-sterilization data 332, number-of-times-of-use data 333, and sterilization treatment data 334. Calibration data 331 refers to data that can specify the calibration parameter defined for each optical element 14 and used by control unit 42. Number-of-times-of-sterilization data 332 refers to data that can specify the number of times of sterilization of probe 10. Sterilization treatment data 334 refers to data that can specify whether or not the sterilization treatment of probe 10 has been performed. Specifically, "1" is stored as sterilization treatment data 334 when the sterilization treatment has been performed, and "0" is stored as sterilization treatment data 334 when the sterilization treatment has not been performed.

The data included in management data 330 is not limited to these pieces of data, and may be a production date of probe 10 or optical element 14, a production lot of probe 10 or optical element 14, a reflectivity (color balance) of optical element 14 for each wavelength, and the like.

Optical element 14 of probe 10 is a component having an individual difference. For example, when optical element 14 is a mirror, each optical element may in some cases have a different reflectivity or a different distortion of a reflected image due to a difference in warpage, refractive index and the like of the optical element caused by, for example, a difference in stress applied to the optical element. Control unit 42 uses the calibration parameter to calibrate the individual difference of an optical element 14A of probe 10A. For example, when a mirror having a reflectivity of 95% is used as a reference in the case of calibrating the difference in reflectivity, control unit 42 performs calibration on a mirror having a reflectivity of 93% so as to increase the luminance by 2% such that the reflectivity increases by 2%.

### [Configuration of Server]

A configuration of server 300 will be described in detail with reference to Fig. 3. Server 300 includes a server storage unit 310 and a server control unit 320. Server storage unit 310 stores management data 330A to management data 330C for each of probe 10A to probe 10C. Management data 330 includes calibration data 331, number-of-times-of-sterilization data 332, number-of-times-of-use data 333, and sterilization treatment data 334.

Server control unit 320 is connected to network 500 and communicates with other computers (e.g., intraoral scanner 200 and sterilizer 400) connected to network 500. When receiving identification data 15 transmitted from the other computer, server control unit 320 searches for management data 330 corresponding to identification data 15 from server storage unit 310, and obtains management data 330 and transmits management data 330 to the other computer. When receiving a command to update management data 330 stored in server storage unit 310 from the other computer, server control unit 320 updates management data 330 stored in server storage unit 310.

### [Configuration of Sterilizer]

Although not shown, sterilizer 400 includes at least a reading unit configured to read identification data 15 of probe 10, a control unit configured to update management data 330 managed for each probe 10, and a sterilizing unit configured to sterilize probe 10.

### [Measurement Process]

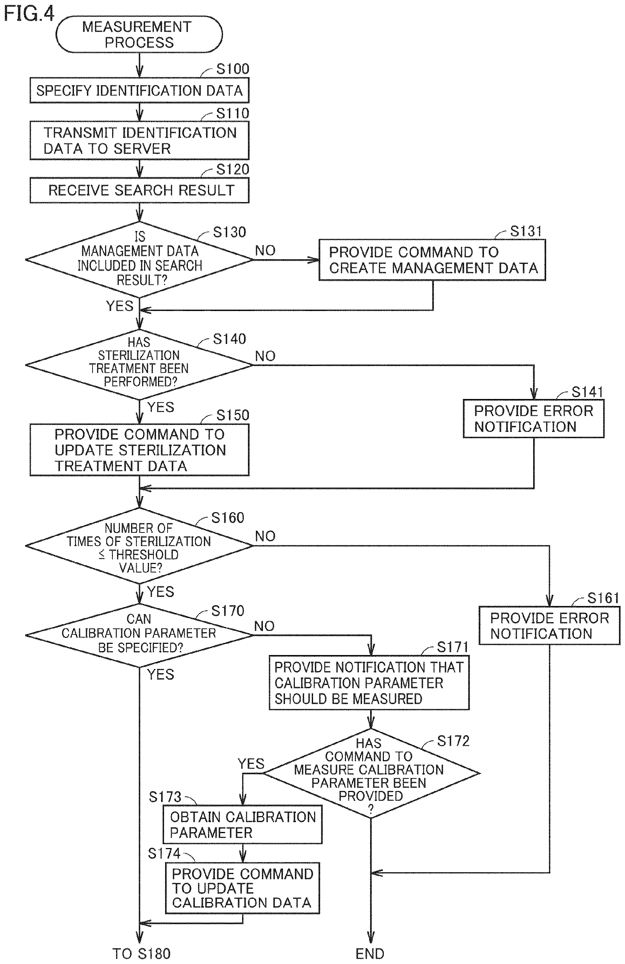

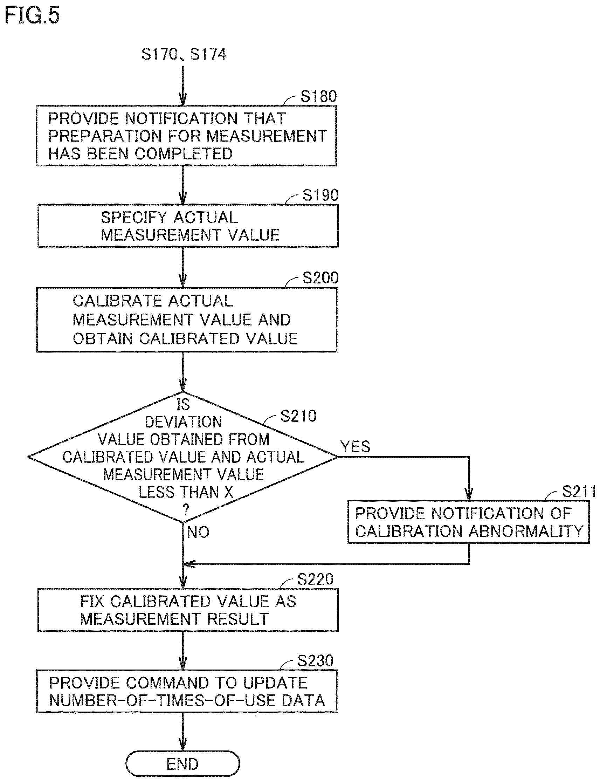

A measurement process executed by control unit 42 of intraoral scanner 200 will be described in detail with reference to Figs. 4 and 5. Figs. 4 and 5 are flowcharts showing the measurement process executed by control unit 42 of intraoral scanner 200 according to the first embodiment of the present invention. A program related to the measurement process is stored in ROM 44 of control unit 42. Control unit 42 executes the measurement process based on detection of execution of an operation for selecting the program related to the measurement process. The operation for selecting the program related to the measurement process may be, for example, an operation for connecting probe 10 to optical measurement unit 30.

In step S100, control unit 42 specifies identification data 15 of probe 10 read by reading unit 32.

In step S110, control unit 42 transmits specified identification data 15 to server 300.

In step S120, control unit 42 receives a search result obtained by server 300 based on transmitted identification data 15. The search result refers to a result of search of the data stored in server storage unit 310 by server control unit 320 based on identification data 15.

When management data 330 corresponding to identification data 15 is stored in server storage unit 310, server control unit 320 transmits management data 330 corresponding to identification data 15 to control device 40. On the other hand, when management data 330 corresponding to identification data 15 is not stored in server storage unit 310, server control unit 320 transmits, to control device 40, information that can specify the absence of management data 330 corresponding to identification data 15.

For example, when probe 10A is connected to optical measurement unit 30, reading unit 32 reads identification data 15A. Control unit 42 specifies identification data 15A read by reading unit 32, and transmits identification data 15A to server 300. When receiving identification data 15A that can specify probe 10A, server control unit 320 determines whether or not management data 330A corresponding to identification data 15A is stored in server storage unit 310. When management data 330A is stored in server storage unit 310, server control unit 320 transmits management data 330A to control device 40 as the search result. On the other hand, when management data 330A is not stored in server storage unit 310, server control unit 320 transmits information that can specify the absence of management data 330A to control device 40 as the search result.

In step S130, control unit 42 determines whether or not management data 330 corresponding to identification data 15 is included in the received search result. That is, control unit 42 determines whether or not management data 330 corresponding to probe 10 connected to intraoral scanner 200 is stored in server storage unit 310. When management data 330 corresponding to identification data 15 is included in the received search result (YES in step S130), the process proceeds to step S140.

When management data 330 corresponding to identification data 15 is not included in the received search result (NO in step S130), control unit 42 commands server 300 to create management data 330 corresponding to identification data 15 in step S131, and then, the process proceeds to step S140. For example, when management data 330A corresponding to identification data 15A is not stored in server storage unit 310, control unit 42 commands server 300 to create management data 330A. As a result, server control unit 320 can register probe 10A with server storage unit 310.

In the case of creating management data 330, server control unit 320 stores predetermined initial values as calibration data 331, number-of-times-of-sterilization data 332, number-of-times-of-use data 333, and sterilization treatment data 334. For example, the initial value of calibration data 331 is data that can specify the absence of the calibration parameter, the initial values of number-of-times-of-sterilization data 332 and number-of-times-of-use data 333 are data that can specify the zero times, and the initial value of sterilization treatment data 334 is data of "0" that can specify that the sterilization treatment has not been performed.

In the first embodiment of the present invention, when management data 330 is not included in the search result, control unit 42 executes the processing in step S140 and the subsequent steps, assuming that the predetermined initial values are stored as management data 330. Control unit 42 may command server 300 to create management data 330 and then obtain management data 330 again.

In step S140, control unit 42 determines whether or not the sterilization treatment has been performed, based on sterilization treatment data 334 included in management data 330.

When the sterilization treatment of probe 10 has not been performed (NO in step S140), control unit 42 provides error notification using notifying unit 34 in step S141, and then, the process proceeds to step S160. The specific content of the error notification is that the sterilization treatment of probe 10 has not been performed. Control unit 42 provides notification that the sterilization treatment has not been performed, by lighting up an LED provided on an outer surface of optical measurement unit 30 as notifying unit 34 and configured to indicate whether or not the sterilization treatment has been performed.

With such a configuration, the measurer can recognize that probe 10 connected to optical measurement unit 30, which is about to be inserted into an oral cavity, is not subjected to sterilization treatment and thus should not be used for measurement. Therefore, it is possible to prevent the measurer from using unsterilized probe 10 by mistake.

After control unit 42 provides the error notification in step S141, the process proceeds to step S160. That is, control unit 42 only provides notification that the sterilization treatment has not been performed, and continues the measurement process. When the sterilization treatment of probe 10 is performed using a sterilizer other than sterilizer 400 communicably connected to server 300, sterilization treatment data 334 may continue to be the information indicating that the sterilization treatment has not been performed, although the sterilization treatment itself has been performed. Even in this case, the error notification is provided in step S141 and then the process proceeds to step S160, and thus, the measurer can continue measurement.

When the sterilization treatment of probe 10 has been performed (YES in step S140), control unit 42 commands server 300 to update sterilization treatment data 334 in step S150. Server control unit 320 of server 300 updates sterilization treatment data 334 included in management data 330 corresponding to identification data 15 to "0".

For example, when probe 10A is used as probe 10, server control unit 320 updates sterilization treatment data 334A included in management data 330A to data of "0" that can specify that the sterilization treatment has not been performed. That is, the information that probe 10A connected to optical measurement unit 30 has been already used is stored in server storage unit 310. When this probe 10A is removed from optical measurement unit 30 and attached to optical measurement unit 30 without being subjected to sterilization treatment, control unit 42 can determine that probe 10A is not subjected to sterilization treatment after being used.

In step S160, control unit 42 determines whether or not the number of times of sterilization is equal to or less than a threshold value. Specifically, by referring to number-of-times-of-sterilization data 332 included in management data 330, control unit 42 specifies the number of times of sterilization of probe 10 and determines whether or not the number of times of sterilization is equal to or less than the threshold value.

The threshold value refers to the number of times that deterioration of optical element 14 caused by the sterilization treatment does not affect measurement. In other words, the determination as to whether or not the number of times of sterilization is equal to or less than the threshold value refers to determination as to whether or not probe 10 can be used for measurement without any problem. Control unit 42 may provide the error notification when the number of times of use, not the number of times of sterilization, is equal to or more than a predetermined threshold value.

When the number of times of sterilization is not equal to or less than the threshold value (NO in step S160), control unit 42 provides error notification using notifying unit 34 in step S161, and ends the measurement process. The specific content of the error notification is that the number of times of sterilization exceeds the threshold value. Control unit 42 provides notification that the sterilization treatment has not been performed, by lighting up an LED provided on the outer surface of optical measurement unit 30 as notifying unit 34 and configured to indicate whether or not the number of times of sterilization exceeds the threshold value. As a result, the measurer can recognize that probe 10 planned to be used cannot be used for measurement. Although control unit 42 provides the error notification when the number of times of sterilization exceeds the threshold value, and then, control unit 42 ends the measurement process, the process may proceed to step S170 in order to continue measurement.

When the number of times of sterilization is equal to or less than the threshold value (YES in step S160), control unit 42 determines whether or not the calibration parameter can be specified in step S170. Specifically, by referring to calibration data 331 included in management data 330, control unit 42 determines whether or not the calibration parameter can be specified, based on whether or not the calibration parameter is registered.

When the calibration parameter cannot be specified (NO in step S170), control unit 42 provides notification that the calibration parameter should be measured using notifying unit 34 in step S171. Control unit 42 provides notification that the calibration parameter should be measured, by lighting up an LED provided on the outer surface of optical measurement unit 30 as notifying unit 34 and configured to indicate whether to measure the calibration parameter or not.

In step S172, control unit 42 determines whether or not a command to measure the calibration parameter has been accepted. Specifically, control unit 42 determines whether or not the measurer has executed an operation for measuring the calibration parameter, based on the information provided by notifying unit 34.

When determining that the command to measure the calibration parameter has not been accepted (NO in step S172), control unit 42 ends the measurement process. The determination as to whether or not the measurement command has been accepted may be awaited for a predetermined time period until the measurement command is accepted, and the process may be ended when the measurement command is not accepted during the time period. Alternatively, the acceptance of the command to measure the calibration parameter may be awaited.

When determining that the command to measure the calibration parameter has been accepted (YES in step S172), control unit 42 obtains the calibration parameter in step S173. In order to obtain the calibration parameter, it is necessary to measure a reference object serving as a reference, and control unit 42 obtains the calibration parameter by measuring the reference object.

For example, in addition to a measurement program for measuring a shape of the object to be measured, control unit 42 includes a calibration parameter measurement program for obtaining the calibration parameter. Intraoral scanner 200 is provided with a selecting unit (not shown) configured to select a program to be executed, of the programs included in control unit 42, and control unit 42 determines the program to be executed, based on an operation of the selecting unit. Control unit 42 determines whether or not the calibration parameter measurement program has been selected by the selecting unit, and when the calibration parameter measurement program has been selected, control unit 42 executes the calibration parameter measurement program and obtains the calibration parameter.

In step S174, control unit 42 transmits the obtained calibration parameter to server 300, and commands server 300 to update calibration data 331. Server control unit 320 of server 300 stores the calibration parameter received as calibration data 331 included in management data 330 corresponding to identification data 15.

For example, when control unit 42 obtains the calibration parameter of probe 10A, control unit 42 transmits the calibration parameter to server 300. Server control unit 320 stores, in server storage unit 310, data that can specify the calibration parameter transmitted from control unit 42 as calibration data 331 included in management data 330A.

After control unit 42 transmits the obtained calibration parameter to server 300 and commands server 300 to update calibration data 331, or when the calibration parameter is registered (YES in step S170), control unit 42 provides notification that preparation for measurement has been completed using notifying unit 34 in step S180 shown in Fig. 5. Control unit 42 indicates the completion of preparation for measurement by providing notification that measurement of the calibration parameter is unnecessary. For example, control unit 42 lights up the LED configured to indicate whether to measure the calibration parameter or not, in a manner different from the manner of lighting up the LED in step S171. Optical measurement unit 30 may include notifying unit 34 configured to indicate that preparation for measurement has been completed, separately from notifying unit 34 configured to indicate whether or not measurement of the calibration parameter is necessary. In such a case, when determining that the sterilization treatment has been performed in step S140 and then providing notification that preparation for measurement has been completed in step S180, control unit 42 may provide notification that measurement of the calibration parameter is unnecessary and separately provide notification that preparation for measurement has been completed.

In step S190, control unit 42 starts measurement. Specifically, control unit 42 specifies the information (actual measurement value) about the reflected light detected by detecting unit 33.

In step S200, control unit 42 calibrates the actual measurement value based on the calibration parameter, and obtains a calibrated value.

In step S210, control unit 42 determines whether or not a deviation value obtained from the actual measurement value and the calibrated value is less than a predetermined value X. When the deviation value is less than X (YES in step S210), control unit 42 moves the process to step S220.

Examples of the deviation value include a deviation value between a change rate of the actual measurement value obtained by dividing the actual measurement value by the calibrated value and a change rate of the actual measurement value when the predetermined reference object is measured at the time of factory shipment. Specifically, when the change rate greatly changes to 50% at the time of measurement although the change rate is 5% at the time of factory shipment, the actual measurement value is estimated to have a problem because the calibration parameter is the same. Since the actual measurement value has a problem, deterioration of optical element 14 or an abnormality of the devices in optical measurement unit 30 are estimated. In this way, in step S210, it is determined whether or not the measurement value has an abnormality.

When the deviation value is equal to or more than X (NO in step S210), control unit 42 provides notification that the calibration has an abnormality using notifying unit 34 in step S211, and then, the process proceeds to step S220. As a result, the measurer can recognize that optical element 14 of probe 10 deteriorates or the devices in optical measurement unit 30 have an abnormality.

In step S220, control unit 42 fixes the calibrated value as a measurement result. When the notification that the calibration has an abnormality is provided in step S211, the measurer may be able to select whether or not to fix the calibrated value as the measurement result. When the notification that the calibration has an abnormality is provided in step S211, control unit 42 may move the process to step S220 in the case of accepting an operation for fixing the calibrated value as the measurement result, and may move the process to step S230 in the case of accepting an operation for not fixing the calibrated value as the measurement result.

In step S230, control unit 42 commands server 300 to update number-of-times-of-use data 333, and ends the measurement process. In response to the command to update number-of-times-of-use data 333, server control unit 320 increments the number of times of use stored in number-of-times-of-use data 333 by 1.

### [Sterilization Information Update Process]

A sterilization information update process executed by sterilizer 400 will be described in detail with reference to Fig. 6. Fig. 6 is a flowchart showing the sterilization information update process executed by a control unit of sterilizer 400 according to the first embodiment of the present invention. For example, the sterilization information update process is executed when probe 10 is subjected to sterilization treatment.

In step S300, the control unit of sterilizer 400 specifies identification data 15 of probe 10 read by a reading unit of sterilizer 400.

In step S310, the control unit of sterilizer 400 commands server 300 to update number-of-times-of-sterilization data 332 and sterilization treatment data 334 included in management data 330 corresponding to identification data 15, and ends the sterilization information update process.

Server control unit 320 of server 300 determines whether or not management data 330 corresponding to identification data 15 is stored. When management data 330 is stored, server control unit 320 of server 300 updates number-of-times-of-sterilization data 332 and sterilization treatment data 334 included in management data 330. Updating number-of-times-of-sterilization data 332 and sterilization treatment data 334 refers to incrementing the number of times of sterilization stored in number-of-times-of-sterilization data 332 by 1 and storing data that can specify that the sterilization treatment has been performed as sterilization treatment data 334.

On the other hand, when management data 330 corresponding to identification data 15 is not stored, server control unit 320 creates management data 330 corresponding to identification data 15. For example, server control unit 320 stores the predetermined initial values as calibration data 331 and number-of-times-of-use data 333, stores one time as number-of-times-of-sterilization data 332, and stores the data that can specify that the sterilization treatment has been performed as sterilization treatment data 334.

As described above, when probe 10 is used and when probe 10 is subjected to sterilization treatment, number-of-times-of-sterilization data 332 and sterilization treatment data 334 are automatically updated (refer to step S150 and step S310). Therefore, the measurer or an administrator of intraoral scanner 200 can easily manage probe 10.

Although the sterilization information update process is executed when probe 10 is subjected to sterilization treatment, the sterilization information update process may be executed when the sterilization treatment is completed. With such a configuration, it is possible to prevent probe 10 during sterilization from being used by mistake.

### [Flow of Calibration Performed by Dental Measurement System]

A flow until calibration of the actual measurement value performed by dental measurement system 100 will be described in detail with reference to Fig. 7. Fig. 7 is a schematic view showing a process executed by dental measurement system 100 according to the first embodiment of the present invention.

In Fig. 7, reading unit 32 reads the shape of opening 11 of probe 10A, thereby specifying identification data 15A of probe 10A. Reading unit 32 reads the two-dimensional code printed on opening 11 of probe 10B, thereby specifying identification data 15B of probe 10B. Reading unit 32 reads the RFID embedded in opening 11 of probe 10C, thereby specifying identification data 15C of probe 10C.

In addition, management data 330A corresponding to probe 10A and management data 330C corresponding to probe 10C are stored in server storage unit 310 of server 300. The data that can specify that the sterilization treatment has not been performed is stored in server storage unit 310 of server 300 as sterilization treatment data 334C included in management data 330C.

When connection unit 31 of optical measurement unit 30 is inserted into opening 11 of probe 10A, reading unit 32 provided in connection unit 31 reads the shape of opening 11. Based on the shape of opening 11 read by reading unit 32, control unit 42 specifies identification data 15A, and transmits identification data 15A to server 300. Server control unit 320 of server 300 transmits management data 330A corresponding to received identification data 15A to control device 40. Control unit 42 calibrates the actual measurement value based on the calibration parameter of calibration data 331A included in management data 330A.

When connection unit 31 of optical measurement unit 30 is inserted into opening 11 of probe 10B, reading unit 32 provided in connection unit 31 reads the two-dimensional code printed on opening 11. Based on the two-dimensional code read by reading unit 32, control unit 42 specifies identification data 15B, and transmits identification data 15B to server 300. Server control unit 320 of server 300 transmits, to control device 40, the information that management data 330B corresponding to received identification data 15B is not stored in server storage unit 310. Control unit 42 specifies that there is no calibration parameter corresponding to probe 10B. The measurer can obtain the calibration parameter of probe 10B by measuring the predetermined reference object. Server 300 stores the calibration parameter of probe 10B obtained by control unit 42 in server storage unit 310 so as to correspond to identification data 15B. Control unit 42 calibrates the actual measurement value based on the obtained calibration parameter of probe 10B.

When connection unit 31 of optical measurement unit 30 is inserted into opening 11 of probe 10C, reading unit 32 provided in connection unit 31 reads the RFID provided in opening 11. Based on the information of the RFID read by reading unit 32, control unit 42 specifies identification data 15C, and transmits identification data 15C to server 300. Server control unit 320 of server 300 transmits management data 330C corresponding to received identification data 15C to control device 40. Based on received management data 330C, control unit 42 determines that measurement using probe 10C is impossible, and provides error notification using notifying unit 34.

In Fig. 7, reading unit 32 of optical measurement unit 30 can read all of the shape, the two-dimensional code and the RFID. However, reading unit 32 of optical measurement unit 30 may only read any one of the shape, the two-dimensional code and the RFID. In addition, when reading unit 32 cannot read identification data 15, control unit 42 may provide notification that identification data 15 cannot be read, using notifying unit 34.

As described above, for use in calibration of the measurement value, intraoral scanner 200 according to the first embodiment of the present invention can obtain management data 330 about the individual difference of probe 10 caused by optical element 14, based on identification data 15 provided on/in probe 10. Therefore, an accurate measurement value can be obtained in consideration of an influence of each individual optical element 14.

Particularly in the case of dental treatment, if a shape of a tooth cannot be accurately measured, it may in some cases be impossible to put a prosthesis into the tooth. Therefore, accurate measurement without any measurement error is desired.

In addition, as shown in step S174 in Fig. 4, intraoral scanner 200 according to the first embodiment of the present invention updates calibration data 331 included in management data 330 corresponding to identification data 15, when the reference object is measured to obtain the calibration parameter. Therefore, even when probe 10 deteriorates as a result of use of probe 10 over a plurality of times, the calibration parameter can be obtained and stored in consideration of the deterioration. Thus, even when probe 10 deteriorates, an accurate measurement value can be obtained.

Although number-of-times-of-use data 333 is not particularly used in intraoral scanner 200 according to the first embodiment of the present invention, notification that a new calibration parameter should be obtained may be provided, for example, when the number of times of use of probe 10 is equal to or more than the predetermined threshold value. As to number-of-times-of-sterilization data 332 as well, although the error notification is provided in the first embodiment of the present invention, notification that a new calibration parameter should be obtained may be provided. In this case, when the new calibration parameter is obtained, control unit 42 may command server 300 to reset or decrement number-of-times-of-use data 333 or number-of-times-of-sterilization data 332.

In addition, as shown in steps S141, S161 and S211 in Fig. 4, intraoral scanner 200 according to the first embodiment of the present invention includes notifying unit 34 configured to be capable of providing notification of the information about probe 10, and can provide error notification using notifying unit 34 when intraoral scanner 200 is in a state where measurement should not be performed. Therefore, it is possible to prevent the measurer from using by mistake probe 10 that should not be used.

In addition, intraoral scanner 200 according to the first embodiment of the present invention is communicably connected to server 300 storing management data 330, and can read management data 330 stored in server 300, based on identification data 15 provided on/in probe 10. Therefore, a storage capacity of RAM 45 can be reduced as compared with the case of storing management data 330 in RAM 45 of intraoral scanner 200.

Management data 330 stored in server 300 according to the first embodiment of the present invention is preferably registered at the time of shipment of probe 10. For example, after probe 10 is formed in a factory, the calibration parameter is measured and registered in the factory. With such a configuration, even when probe 10 is used for the first time after shipment from the factory, measurement of the calibration parameter is unnecessary and measurement can be started immediately.

Although management data 330 includes calibration data 331, number-of-times-of-sterilization data 332, number-of-times-of-use data 333, and sterilization treatment data 334, management data 330 may only include at least calibration data 331. In addition, although sterilization treatment data 334, number-of-times-of-use data 333, number-of-times-of-sterilization data 332, and calibration data 331 are stored when management data 330 corresponding to probe 10 is stored, only management data 330 may be stored, and if there is no data to be updated at the time of updating the respective pieces of data, the respective pieces of data may be stored.

In the first embodiment of the present invention, in determination as to whether or not the command to measure the calibration parameter has been accepted, control unit 42 determines whether or not the measurer has performed the operation for measuring the calibration parameter, based on the information provided by notifying unit 34. However, in the case of obtaining the calibration parameter by measuring the predetermined reference object as a method for measuring the calibration parameter, identification data that can specify the reference object may be provided on the reference object and reading unit 32 may read the identification data, thereby determining that the command to measure the calibration parameter has been accepted.

In addition, in the first embodiment of the present invention, when accepting the command to measure the calibration parameter and obtaining the calibration parameter, control unit 42 commands server 300 to update calibration data 331. However, control unit 42 may command server 300 to update calibration data 331 whenever the new calibration parameter is obtained. In addition, when the change rate is equal to or more than the threshold value as a result of comparison between the obtained calibration parameter and the newly obtained parameter, control unit 42 may provide notification that the newly obtained parameter has an abnormality. In addition, control unit 42 may compare the newly obtained parameter with a predefined value, not the obtained calibration parameter.

Although dental measurement system 100 is provided with control device 40 in the first embodiment, optical measurement unit 30 may be provided with a communication unit configured to allow communication with server 300 via network 500 and server 300 may be provided with the function of control unit 42 of control device 40. With such a configuration, a processing burden of intraoral scanner 200 can be reduced.

In addition, in the first embodiment, sterilization treatment data 334 is provided. However, without providing sterilization treatment data 334, it may be determined that the sterilization treatment has not been performed, when the number of times of use is larger than or equal to the number of times of sterilization as a result of comparison between number-of-times-of-sterilization data 332 and number-of-times-of-use data 333.

Although dental measurement system 100 includes intraoral scanner 200, server 300, sterilizer 400, and network 500 in the first embodiment, accurate measurement can be performed as long as dental measurement system 100 includes at least intraoral scanner 200 and server 300 that can communicate with intraoral scanner 200. A method for communication between intraoral scanner 200 and server 300 is not limited to communication via network 500 such as the Internet, and intraoral scanner 200 and server 300 may be connected such that intraoral scanner 200 and server 300 can directly communicate with each other. Alternatively, at least one of control device 40 and optical measurement unit 30 may participate in a peer-to-peer management information sharing network.

Although notifying unit 34 is the LED in the first embodiment, notifying unit 34 may be a speaker or a liquid crystal panel that can display an image. When the LED or the liquid crystal panel is used as notifying unit 34, only the measurer can be notified of the occurrence of the error, without notifying the subject such as the patient of the occurrence of the error. For example, when the sterilization treatment of probe 10 is performed using a sterilizer other than sterilizer 400 communicably connected to server 300, and thus, the sterilization treatment itself is performed although sterilization treatment data 334 is not updated, measurement proceeds with error notification being provided. When the subject can recognize the occurrence of the error, the subject has a discomfort feeling against insertion of the measurement instrument having the error into his/her oral cavity, although probe 10 subjected to sterilization treatment is inserted into the oral cavity. In order to address this, the subject is not notified of the occurrence of the error. With such a configuration, the subject never has an unnecessary discomfort feeling when the error notification is provided although probe 10 subjected to sterilization treatment is used.

In addition, when abnormality notification is provided in steps S141, S161 and S211, control unit 42 may provide the notification over a prescribed time period and then stop the notification, or may stop the notification based on acceptance of a command to stop the notification.

### (Second Embodiment)

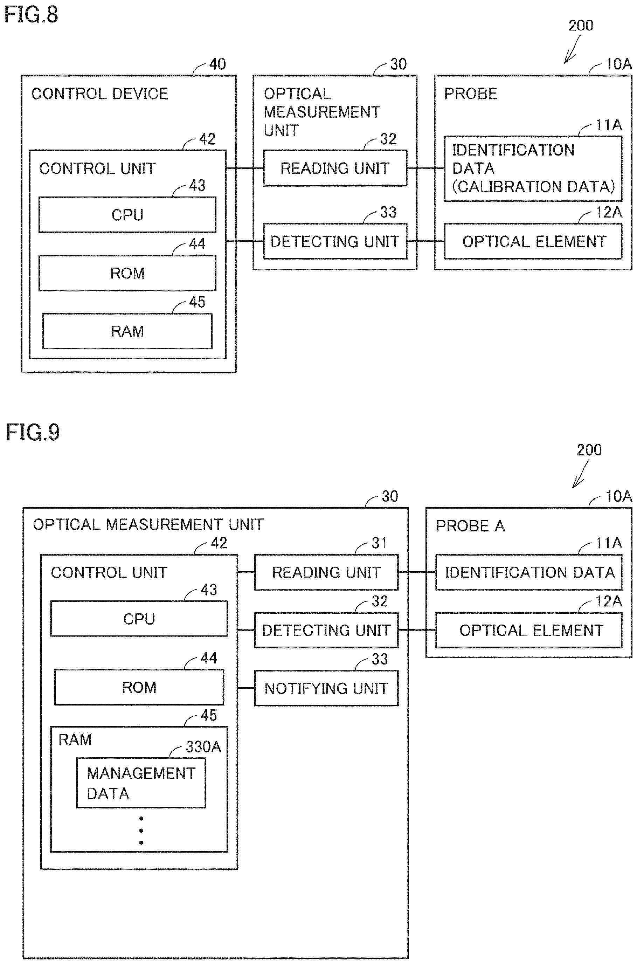

In dental measurement system 100 according to the first embodiment, the calibration parameter is stored in server storage unit 310 as calibration data 331. However, in dental measurement system 100 according to a second embodiment, probe 10 is provided with the calibration parameter as identification data 15, and this configuration will be described. Fig. 8 is a block diagram for illustrating intraoral scanner 200 according to the second embodiment of the present invention. In dental measurement system 100 according to the second embodiment, the same components as those of dental measurement system 100 according to the first embodiment shown in Fig. 3 are denoted by the same reference characters and detailed description will not be repeated.

Dental measurement system 100 according to the second embodiment includes intraoral scanner 200. Probe 10 is provided with calibration data 331 including the calibration parameter. Reading unit 32 of optical measurement unit 30 reads calibration data 331, and thereby, control unit 42 can specify the calibration parameter of probe 10. Using the calibration parameter, control unit 42 calibrates a measurement distortion caused by optical element 14 of probe 10.

Identification data 15 according to the second embodiment may be the calibration parameter itself related to probe 10, or may be information that can be converted to the calibration parameter based on a predetermined conversion formula.

Since identification data 15 provided for each probe 10 is the calibration parameter itself as described above, a measurement distortion caused by optical element 14 of probe 10 can be calibrated without including server 300 and network 500 separately from intraoral scanner 200.

In the second embodiment, probe 10 is provided with the calibration parameter as identification data 15. However, probe 10 may be provided with a rewritable storage unit and management data 330 for managing probe 10, such as calibration data 331, number-of-times-of-sterilization data 332, number-of-times-of-use data 333, and sterilization treatment data 334 may be registered with the storage unit.

### (Third Embodiment)

In dental measurement system 100 according to the first embodiment, management data 330 is stored in server storage unit 310, and based on management data 330, control unit 42 calibrates a measurement distortion caused by optical element 14 of probe 10. However, in dental measurement system 100 according to a third embodiment, optical measurement unit 30 has the function of control device 40 and the function of server storage unit 310, and this configuration will be described. Fig. 9 is a block diagram for illustrating intraoral scanner 200 according to the third embodiment of the present invention. In dental measurement system 100 according to the third embodiment, the same components as those of dental measurement system 100 according to the first embodiment shown in Fig. 3 are denoted by the same reference characters and detailed description will not be repeated.

Dental measurement system 100 according to the third embodiment includes intraoral scanner 200. Intraoral scanner 200 includes optical measurement unit 30 and probe 10. Optical measurement unit 30 includes control unit 42. RAM 45 of control unit 42 of optical measurement unit 30 stores management data 330 for each probe 10, such as management data 330A.

Since optical measurement unit 30 includes control unit 42 as described above, dental measurement system 100 can be used even under the environment where the Internet is inaccessible. In addition, since optical measurement unit 30 includes control unit 42, there is no need to provide control device 40 and thus intraoral scanner 200 can be reduced in size.

Control device 40 may be provided and management data 330 may be stored in RAM 45 of control device 40. With such a configuration, optical measurement unit 30, which is the component gripped by the measurer, can be reduced in size.

In addition, when a command to update management data 330 is received from outside intraoral scanner 200 such as a manufacturer of probe 10 in the case where management data 330 is stored in control device 40 or optical measurement unit 30, control unit 42 may update management data 330. With such a configuration, the information of management data 330 can be constantly updated to the latest data, and thus, more accurate measurement can be performed.

### (Fourth Embodiment)

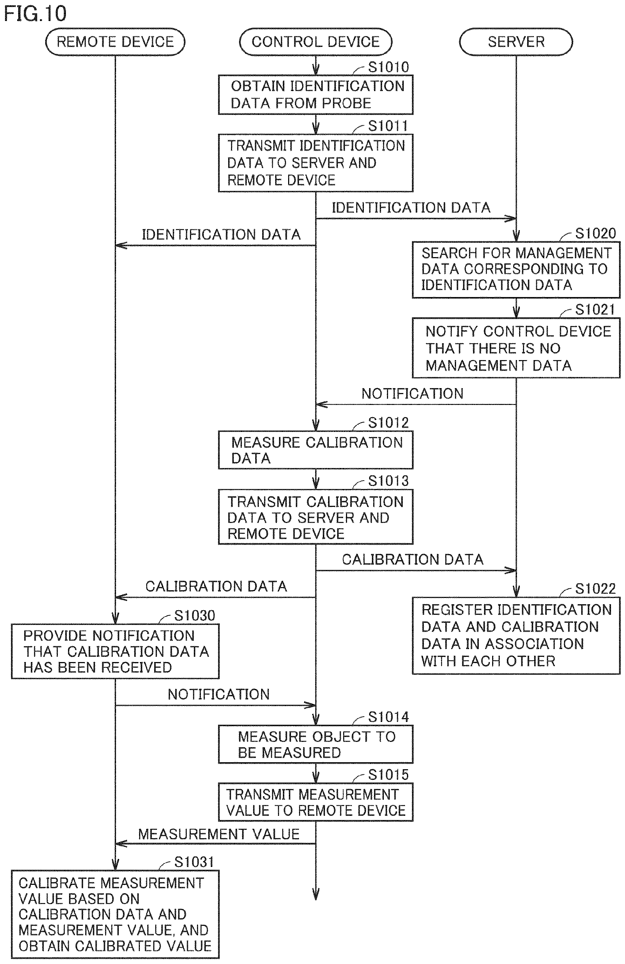

In dental measurement system 100 according to the first embodiment, control device 40 of intraoral scanner 200 performs calibration of the data using calibration data 331, provision of the command to update the data, and the like. However, in dental measurement system 100 according to a fourth embodiment, the process about calibration executed by control device 40 may be executed by a remote device 600 different from intraoral scanner 200. Fig. 10 is one example of a flowchart showing a process executed by dental measurement system 100 according to the fourth embodiment of the present invention, when there is no management data 330 corresponding to identification data 15.

In step S1010, control device 40 obtains identification data 15 from probe 10.

In step S1011, control device 40 transmits identification data 15 to server 300 and remote device 600.

In step S1020, server control unit 320 of server 300 having received identification data 15 searches for management data 330 corresponding to identification data 15.

In step S1021, server control unit 320 transmits the search result in step S1020 to control device 40. Specifically, server control unit 320 transmits, to control device 40, data that can specify that there is no management data 330.

Control device 40 having received the search result executes the process corresponding to the search result. Specifically, control device 40 measures calibration data 331 in step S1012, and transmits calibration data 331 to server 300 and remote device 600 in step S1013. After transmitting calibration data 331 to server 300, control device 40 may return to step S1011 and receive calibration data 331 registered with server 300, and then, transmit calibration data 331 to remote device 600. Since control device 40 transmits calibration data 331 to server 300 and remote device 600 in step S1013, a processing burden of control device 40 and server 300 can be reduced.

In step S1022, server control unit 320 of server 300 having received calibration data 331 registers identification data 15 and calibration data 331 in association with each other.

In step S1030, remote device 600 notifies control device 40 that calibration data 331 has been received, and commands control device 40 to start measurement of an object to be measured.

In step S1014, control device 40 having received calibration data 331 measures the object to be measured.

In step S1015, control device 40 transmits the measurement value to remote device 600.

In step S1031, remote device 600 calibrates the measurement value based on calibration data 331 received in step S1030 and the received measurement value, and obtains a calibrated value.

Although remote device 600 and server 300 are different components in the fourth embodiment, server control unit 320 may include a calibration program for calibrating the measurement value and server 300 may calibrate the measurement value.

Since remote device 600 different from intraoral scanner 200 executes the process about calibration as described above, a storage capacity of control unit 42 of intraoral scanner 200 can be reduced.

### [Modification]

Although optical element 14 according to the first to fourth embodiments is the mirror, optical element 14 may be a lens, a prism or the like. In addition, although probe 10 includes one mirror as optical element 14, probe 10 may include a plurality of optical elements 14 of one type, or may include a plurality of optical elements 14 of different types. When probe 10 includes a lens or a prism as optical element 14, a refractive index of optical element 14 and a spectral transmission factor of optical element 14 may be included as management data 330. When probe 10 includes a plurality of optical elements 14, the actual measurement value is calibrated in consideration of all optical elements 14.

In addition, although reading unit 32 is provided in connection unit 31 of optical measurement unit 30 in the first to fourth embodiments, reading unit 32 may be provided in control device 40, or may be provided by another reader. When another reader is provided, dental measurement system 100 of the present invention is also applicable to intraoral scanner 200 that does not include reading unit 32.

Although notifying unit 34 is provided in optical measurement unit 30 in the first embodiment, notifying unit 34 may be provided in probe 10, control device 40, another computer that can communicate with a control unit configured to provide a notification command, or the like.

In addition, although no mention is made of whether or not the actual measurement value and the calibrated value are stored as management data 330 in the first to fourth embodiments, the actual measurement value and the calibrated value may be stored as management data 330 in association with the number of times of use and the number of times of sterilization. With such a configuration, data for analyzing an influence of the number of times of sterilization and the number of times of use on the optical element can be stored.

In addition, although identification data 15 is provided so as to be capable of identifying probe 10 individually in the first to fourth embodiments, identification data 15 may be provided so as to be capable of identifying a prescribed group, for example. Specifically, when a large-sized optical element is cut into small pieces to thereby obtain optical elements 14 for probes 10, probes 10 belonging to the same optical element may be grouped into the same group. With such a configuration, an amount of management data 330 can be reduced.

In addition, although intraoral scanner 200 can measure the calibration parameter of probe 10 in the first to fourth embodiments, intraoral scanner 200 does not necessarily need to have such a function. In addition, although intraoral scanner 200 can update calibration data 331, number-of-times-of-sterilization data 332, number-of-times-of-use data 333, or sterilization treatment data 334, intraoral scanner 200 does not necessarily need to have the update function.

In addition, although server 300 and control device 40 have the function of managing management data 330 in the first embodiment, control device 40 may have the entire function of managing management data 330. Alternatively, server 300 may have the entire function of managing management data 330. Alternatively, optical measurement unit 30 may have the entire function of managing management data 330.

In addition, although no mention is made of the timing of registration of management data 330 in the first to fourth embodiments, management data 330 may be registered before shipment of probe 10. With such a configuration, when the user uses probe 10 for the first time, the user can immediately start accurate measurement without the work of measuring the calibration parameter, and thus, the convenience of probe 10 is improved.

In addition, although management data 330 is updated during measurement or during sterilization, management data 330 may be updated when the administrator of probe 10, such as the measurer or the manufacturer, operates the device communicably connected to the device including the storage unit having management data 330 stored thereon. For example, when the sterilization treatment is performed using a sterilizer that is not included in dental measurement system 100, a person who has performed the sterilization treatment may manually update the number of times of sterilization.

In addition, management data 330 of probe 10 may be updated by the manufacturer after shipment of probe 10. For example, when the calibration parameter must be updated in response to maintenance of optical measurement unit 30 or control device 40 of intraoral scanner 200, a person who has performed maintenance may update management data 330. With such a configuration, when intraoral scanner 200 is used after maintenance, intraoral scanner 200 can be used immediately, and thus, the convenience of dental measurement system 100 is improved.

It should be understood that the embodiments disclosed herein are illustrative and non-restrictive in every respect. The scope of the present invention is defined by the terms of the claims.

### REFERENCE SIGNS LIST

10, 10A, 10B, 10C probe; 11 opening; 12 housing; 13 measurement window; 14, 14A optical element; 15, 15A, 15B, 15C identification data; 30 optical measurement unit; 31 connection unit; 32 reading unit; 33 detecting unit; 34 notifying unit; 40 control device; 41 communication unit; 42 control unit; 43 CPU; 44 ROM; 45 RAM; 100 dental measurement system; 200 intraoral scanner; 300 server; 310 server storage unit; 320 server control unit; 330, 330A, 330B, 330C management data; 331, 331A calibration data; 332 number-of-times-of-sterilization data; 333 number-of-times-of-use data; 334, 334A, 334C sterilization treatment data; 400 sterilizer; 500 network; 600 remote device.

## Claims

1. A dental measurement apparatus (200) comprising:
a main body unit (30) configured to measure an object to be measured; and
a tip unit (10) provided so as to be attachable and detachable to and from the main body unit (30),
the tip unit (10) including:
an optical element (14) configured to be capable of introducing light from the object to be measured into the main body unit (30) for measurement in the main body unit (30); and
identification information (15) for identifying the tip unit (10) individually, wherein
the main body unit (30) is configured to obtain management information about an individual difference of the tip unit caused by the optical element, based on the identification information, and to use the management information for use in calibration in the dental measurement apparatus (200); and
the main body unit (30) is configured to use the management information to improve the accuracy of the measurement in the main body unit (30).

2. The dental measurement apparatus (200) according to claim 1, wherein
the main body unit (30) includes a reading unit (32) configured to read the identification information (15) of the tip unit, and
the reading unit (32) is configured to read the identification information (15) from a shape of at least a part of the tip unit (10).

3. The dental measurement apparatus (200) according to claim 1, wherein
the main body unit (30) includes a reading unit (32) configured to read the identification information (15) of the tip unit (10), and
the reading unit (32) is configured to read the identification information (15) by optically recognizing a tag provided on the tip unit (10).

4. The dental measurement apparatus (200) according to claim 1, wherein
the main body unit (30) includes a reading unit (32) configured to read the identification information (15) of the tip unit (10), and
the reading unit (32) is configured to read the identification information (15) by electromagnetically recognizing a tag provided in the tip unit (10).

5. The dental measurement apparatus (200) according to any one of claims 2 to 4, wherein
the main body unit (30) is provided with the reading unit (32) in a portion connected to the tip unit (10), and
the reading unit (32) is configured to read the identification information (15) when the main body unit (30) and the tip unit (10) are connected to each other.

6. The dental measurement apparatus (200) according to any one of claims 1 to 5, wherein
the main body unit (30) includes an update process unit configured to update the management information based on obtained individual difference information of the optical element (14), when measuring a reference object instead of the object to be measured.

7. The dental measurement apparatus (200) according to claim 6, wherein
the main body unit (30) includes a notifying unit (34) configured to be capable of providing notification of information about the tip unit (10), and
the notifying unit (34) is configured to compare the obtained individual difference information with the management information, and to provide notification when a deviation value between the information exceeds a threshold value.

8. The dental measurement apparatus (200) according to any one of claims 1 to 7, wherein
the main body unit (30) can be connected to a management apparatus (300) configured to store the management information, and
the main body unit (30) can read the management information stored in the management apparatus (300), based on the identification information (15) of the tip unit (10).

9. The dental measurement apparatus (200) according to any one of claims 1 to 7, wherein
the main body unit (30) includes a storage unit configured to store the management information, and
the main body unit (30) can read the management information stored in the storage unit, based on the identification information (15) of the tip unit (10).

10. The dental measurement apparatus (200) according to claim 9, wherein the main body unit (30) includes a rewriting unit configured to rewrite the management information stored in the storage unit, when newly obtaining the management information from outside.

11. A dental measurement system (100) comprising: a dental measurement apparatus (200) according to claim 1 and a management apparatus (300) configured to manage management information used in calibration in the dental measurement apparatus (200)
the management apparatus (300) including
a storage unit (310) configured to store an individual difference of the tip unit (10) caused by the optical element (14) as the management information in association with the identification information.

12. The dental measurement system (100) according to claim 11, wherein
the management apparatus (300) is configured to store the management information in the storage unit (310) before shipment of the tip unit (10).

## Patentansprüche

1. Dentalmessvorrichtung (200), aufweisend:
eine Hauptkörpereinheit (30), die dafür konfiguriert ist, ein zu messendes Objekt zu messen; und
eine Aufsatzeinheit (10), die so vorgesehen ist, dass sie an der Hauptkörpereinheit (30) anbringbar und von ihr abnehmbar ist,
wobei die Aufsatzeinheit (10) umfasst:
ein optisches Element (14), das dafür konfiguriert ist, Licht von dem zu messenden Objekt in die Hauptkörpereinheit (30) für eine Messung in der Hauptkörpereinheit (30) einleiten zu können; und
eine Identifizierungsinformation (15), um die Aufsatzeinheit (10) individuell zu identifizieren, wobei
die Hauptkörpereinheit (30) dafür konfiguriert ist, eine Verwaltungsinformation über einen durch das optische Element bedingten individuellen Unterschied der Aufsatzeinheit basierend auf der Identifizierungsinformation zu erhalten und die Verwaltungsinformation zur Verwendung bei einer Kalibrierung in der Dentalmessvorrichtung (200) zu verwenden; und
die Hauptkörpereinheit (30) dafür konfiguriert ist, die Verwaltungsinformation zu verwenden, um die Genauigkeit der Messung in der Hauptkörpereinheit (30) zu verbessern.

2. Dentalmessvorrichtung (200) nach Anspruch 1, wobei
die Hauptkörpereinheit (30) eine Leseeinheit (32) enthält, die dafür konfiguriert ist, die Identifizierungsinformation (15) der Aufsatzeinheit zu lesen, und
die Leseeinheit (32) dafür konfiguriert ist, die Identifizierungsinformation (15) aus einer Form von zumindest einem Teil der Aufsatzeinheit (10) zu lesen.

3. Dentalmessvorrichtung (200) nach Anspruch 1, wobei
die Hauptkörpereinheit (30) eine Leseeinheit (32) enthält, die dafür konfiguriert ist, die Identifizierungsinformation (15) der Aufsatzeinheit (10) zu lesen, und
die Leseeinheit (32) dafür konfiguriert ist, die Identifizierungsinformation (15) durch optisches Erkennen einer auf der Aufsatzeinheit (10) vorgesehenen Kennzeichnung zu lesen.

4. Dentalmessvorrichtung (200) nach Anspruch 1, wobei
die Hauptkörpereinheit (30) eine Leseeinheit (32) enthält, die dafür konfiguriert ist, die Identifizierungsinformation (15) der Aufsatzeinheit (10) zu lesen, und
die Leseeinheit (32) dafür konfiguriert ist, die Identifizierungsinformation (15) durch elektromagnetisches Erkennen einer in der Aufsatzeinheit (10) vorgesehenen Kennzeichnung zu lesen.

5. Dentalmessvorrichtung (200) nach einem der Ansprüche 2 bis 4, wobei
die Hauptkörpereinheit (30) mit der Leseeinheit (32) in einem mit der Aufsatzeinheit (10) verbundenen Teil versehen ist und
die Leseeinheit (32) dafür konfiguriert ist, die Identifizierungsinformation (15) zu lesen, wenn die Hauptkörpereinheit (30) und die Aufsatzeinheit (10) miteinander verbunden sind.

6. Dentalmessvorrichtung (200) nach einem der Ansprüche 1 bis 5, wobei
die Hauptkörpereinheit (30) eine Einheit für einen Aktualisierungsprozess enthält, die dafür konfiguriert ist, basierend auf einer erhaltenen Information über einen individuellen Unterschied des optischen Elements (14) zu aktualisieren, wenn anstelle des zu messenden Objekts ein Referenzobjekt gemessen wird.

7. Dentalmessvorrichtung (200) nach Anspruch 6, wobei
die Hauptkörpereinheit (30) eine Benachrichtigungseinheit (34) enthält, die dafür konfiguriert ist, eine Benachrichtigung der Information über die Aufsatzeinheit (10) liefern zu können, und
die Benachrichtigungseinheit (34) dafür konfiguriert ist, die erhaltene Information über einen individuellen Unterschied mit der Verwaltungsinformation zu vergleichen und eine Benachrichtigung zu liefern, wenn ein Abweichungswert zwischen den Informationen einen Schwellenwert übersteigt.

8. Dentalmessvorrichtung (200) nach einem der Ansprüche 1 bis 7, wobei
die Hauptkörpereinheit (30) mit einer Verwaltungsvorrichtung (300) verbunden werden kann, die dafür konfiguriert ist, die Verwaltungsinformation zu speichern, und
die Hauptkörpereinheit (30) die in der Verwaltungsvorrichtung (300) gespeicherte Verwaltungsinformation basierend auf der Identifizierungsinformation (15) der Aufsatzeinheit (10) lesen kann.

9. Dentalmessvorrichtung (200) nach einem der Ansprüche 1 bis 7, wobei
die Hauptkörpereinheit (30) eine Speichereinheit enthält, die dafür konfiguriert ist, die Verwaltungsinformation zu speichern, und
die Hauptkörpereinheit (30) die in der Speichereinheit gespeicherte Verwaltungsinformation basierend auf der Identifizierungsinformation (15) der Aufsatzeinheit (10) lesen kann.

10. Dentalmessvorrichtung (200) nach Anspruch 9, wobei
die Hauptkörpereinheit (30) eine Umschreibeinheit enthält, die dafür konfiguriert ist, die in der Speichereinheit gespeicherte Verwaltungsinformation umzuschreiben, wenn die Verwaltungsinformation von außen neu erhalten wird.

11. Dentalmesssystem (100), aufweisend: eine Dentalmessvorrichtung (200) nach Anspruch 1 und eine Verwaltungsvorrichtung (300), die dafür konfiguriert ist, eine bei einer Kalibrierung in der Dentalmessvorrichtung (200) verwendete Verwaltungsinformation zu verwalten, wobei
die Verwaltungseinheit (300) umfasst:
eine Speichereinheit (310), die dafür konfiguriert ist, einen durch das optische Element (14) bedingten individuellen Unterschied der Aufsatzeinheit (10) als die Verwaltungsinformation in Verbindung mit der Identifizierungsinformation zu speichern.

12. Dentalmesssystem (100) nach Anspruch 11, wobei
die Verwaltungsvorrichtung (300) dafür konfiguriert ist, die Verwaltungsinformation in der Speichereinheit (310) vor einem Versand der Aufsatzeinheit (10) zu speichern.

## Revendications

1. Appareil de mesure dentaire (200) comprenant :
une unité de corps principale (30) configurée pour mesurer un objet à mesurer ; et
une unité de pointe (10) prévue de manière à pouvoir être attachée à l'unité de corps principale (30) ou détachée de celle-ci,
l'unité de pointe (10) incluant :
un élément optique (14) configuré pour être capable d'introduire de la lumière depuis l'objet à mesurer dans l'unité de corps principale (30) pour une mesure dans l'unité de corps principale (30) ; et
des informations d'identification (15) pour identifier l'unité de pointe (10) individuellement, dans lequel
l'unité de corps principale (30) est configurée pour obtenir des informations de gestion sur une différence individuelle de l'unité de pointe causée par l'élément optique, sur la base des informations d'identification, et pour utiliser les informations de gestion pour une utilisation dans l'étalonnage dans l'appareil de mesure dentaire (200) ; et
l'unité de corps principale (30) est configurée pour utiliser les informations de gestion pour améliorer la précision de la mesure dans l'unité de corps principale (30).

2. Appareil de mesure dentaire (200) selon la revendication 1, dans lequel
l'unité de corps principale (30) inclut une unité de lecture (32) configurée pour lire les informations d'identification (15) de l'unité de pointe, et
l'unité de lecture (32) est configurée pour lire les informations d'identification (15) depuis une forme d'une partie au moins de l'unité de pointe (10).

3. Appareil de mesure dentaire (200) selon la revendication 1, dans lequel
l'unité de corps principale (30) inclut une unité de lecture (32) configurée pour lire les informations d'identification (15) de l'unité de pointe (10), et
l'unité de lecture (32) est configurée pour lire les informations d'identification (15) par reconnaissance optique d'un indicateur prévu sur l'unité de pointe (10).

4. Appareil de mesure dentaire (200) selon la revendication 1, dans lequel
l'unité de corps principale (30) inclut une unité de lecture (32) configurée pour lire les informations d'identification (15) de l'unité de pointe (10), et
l'unité de lecture (32) est configurée pour lire les informations d'identification (15) par reconnaissance électromagnétique d'un indicateur prévu sur l'unité de pointe (10).

5. Appareil de mesure dentaire (200) selon l'une quelconque des revendications 2 à 4, dans lequel
l'unité de corps principale (30) est pourvue de l'unité de lecture (32) dans une partie reliée à l'unité de pointe (10), et
l'unité de lecture (32) est configurée pour lire les informations d'identification (15) quand l'unité de corps principale (30) et l'unité de pointe (10) sont reliées l'une à l'autre.

6. Appareil de mesure dentaire (200) selon l'une quelconque des revendications 1 à 5, dans lequel
l'unité de corps principale (30) inclut une unité de traitement d'actualisation configurée pour actualiser les informations de gestion sur la base d'informations de différence individuelle de l'élément optique (14) obtenues, lors de la mesure d'un objet de référence à la place de l'objet à mesurer.

7. Appareil de mesure dentaire (200) selon la revendication 6, dans lequel
l'unité de corps principale (30) inclut une unité de notification (34) configurée pour être capable de fournir une notification d'informations sur l'unité de pointe (10), et
l'unité de notification (34) est configurée pour comparer les informations de différence individuelle obtenues aux informations de gestion, et pour fournir une notification quand une valeur d'écart entre les informations dépasse une valeur seuil.

8. Appareil de mesure dentaire (200) selon l'une quelconque des revendications 1 à 7, dans lequel
l'unité de corps principale (30) peut être reliée à un appareil de gestion (300) configuré pour stocker les informations de gestion, et
l'unité de corps principale (30) peut lire les informations de gestion stockées dans l'appareil de gestion (300), sur la base des informations d'identification (15) de l'unité de pointe (10).

9. Appareil de mesure dentaire (200) selon l'une quelconque des revendications 1 à 7, dans lequel
l'unité de corps principale (30) inclut une unité de stockage configurée pour stocker les informations de gestion, et
l'unité de corps principale (30) peut lire les informations de gestion stockées dans l'unité de stockage, sur la base des informations d'identification (15) de l'unité de pointe (10).

10. Appareil de mesure dentaire (200) selon la revendication 9, dans lequel l'unité de corps principale (30) inclut une unité de réécriture configurée pour réécrire les informations de gestion stockées dans l'unité de stockage, lorsque des informations de gestion sont nouvellement obtenues depuis l'extérieur.

11. Système de mesure dentaire (100) comprenant : un appareil de mesure dentaire (200) selon la revendication 1 et un appareil de gestion (300) configuré pour gérer des informations de gestion utilisées dans l'étalonnage dans l'appareil de mesure dentaire (200)
l'appareil de gestion (300) incluant
une unité de stockage (310) configurée pour stocker une différence individuelle de l'unité de pointe (10) causée par l'élément optique (14) comme informations de gestion en association avec les informations d'identification.

12. Système de mesure dentaire (100) selon la revendication 11, dans lequel l'appareil de gestion (300) est configuré pour stocker les informations de gestion dans l'unité de stockage (310) avant l'expédition de l'unité de pointe (10).
